# EUROPEAN PATENT SPECIFICATION

(11) **EP 3 409 293 B1**
(45) Date of publication and mention of the grant of the patent: **24.11.2021**
(21) Application number: 18174611.6
(22) Date of filing: 28.05.2018
(51) Int. Cl.: A61K 47/36, A61K 36/87, A61K 9/127, A61K 47/22, A61K 47/32, A61K 47/38

(54) **COMPLEXES COMPRISING PHOSPHOLIPIDS, WATER-SOLUBLE DEXTRIN FIBERS AND ANTIOXIDANT ACTIVE PRINCIPLES OF NATURAL ORIGIN, THEIR PREPARATION AND USE IN ORAL FORMULATIONS**
KOMPLEXE UMFASSEND PHOSPHOLIPIDE, WASSERLÖSLISCHE DEXTRINFASERN UND ANTIOXIDANSWIRKSTOFFE NATÜRLICHEN URSPRUNGS, IHRE HERSTELLUNG UND VERWENDUNG IN ORALEN FORMULIERUNGEN
COMPLEXES COMPRENANT DES PHOSPHOLIPIDES, DES FIBRES DE DEXTRINES SOLUBLES DANS L'EAU ET DES PRINCIPES ACTIFS ANTIOXYDANTS D'ORIGINE NATURELLE, LEUR PRÉPARATION ET UTILISATION DANS DES FORMULATIONS ORALES

(30) Priority: 31.05.2017 IT 201700059875
(43) Date of publication of application: 05.12.2018
(73) Proprietor: UNIVERSITA' DEGLI STUDI DI CAGLIARI, 09124 Cagliari (IT)
(72) Inventor: MANCONI, Maria, 09124 Cagliari (IT); Manca, Maria Letizia, 09124 Cagliari (IT); CADDEO, Carla, 09124 Cagliari (IT); Fadda, Anna Maria, 09124 Cagliari (IT)
(74) Representative: Primiceri, Maria Vittoria

(56) References cited:
- WO-A1-2011/095938
- CN-B- 102 580 111
- INES CASTANGIA ET AL: "Therapeutic efficacy of quercetin enzyme-responsive nanovesicles for the treatment of experimental colitis in rats", ACTA BIOMATERIALIA, vol. 13, 18 November 2014 (2014-11-18), pages 216-227, XP055454066, AMSTERDAM, NL ISSN: 1742-7061, DOI: 10.1016/j.actbio.2014.11.017
- SANTOSH S. DHULE ET AL: "Curcumin-loaded [gamma]-cyclodextrin liposomal nanoparticles as delivery vehicles for osteosarcoma", NANOMEDICINE: NANOTECHNOLOGY, BIOLOGY AND MEDICINE, vol. 8, no. 4, May 2012 (2012-05), pages 440-451, XP055365268, NL ISSN: 1549-9634, DOI: 10.1016/j.nano.2011.07.011
- CATALAN-LATORRE ANA ET AL: "Freeze-dried eudragit-hyaluronan multicompartment liposomes to improve the intestinal bioavailability of curcumin", EUROPEAN JOURNAL OF PHARMACEUTICS AND BIOPHARMACEUTICS, ELSEVIER SCIENCE PUBLISHERS B.V., AMSTERDAM, NL, vol. 107, 25 June 2016 (2016-06-25), pages 49-55, XP029720434, ISSN: 0939-6411, DOI: 10.1016/J.EJPB.2016.06.016
- KATA TUZA ET AL: "Synthesis of modified cyclic and acyclic dextrins and comparison of their complexation ability", BEILSTEIN JOURNAL OF ORGANIC CHEMISTRY, vol. 10, 14 November 2014 (2014-11-14), pages 2836-2843, XP055748986, DOI: 10.3762/bjoc.10.301

## Description

### Technical Field and Summary of the Invention

The present invention relates to the obtainment of complexes formed by phospholipids, water-soluble dextrin fibers and antioxidizing substances of natural origin forming lamellar vesicles, hereafter referred to as *nutriosomes,* which are intended for the formulation of nutraceutical and/or pharmaceutical products for oral use in the treatment of gastrointestinal dysbiosis.

More specifically, the present invention relates to the preparation and application of dispersions of said nutriosomes in substantially aqueous solvents; to the lyophilization thereof for obtaining a solid and stable complex formed by phospholipids, dextrin fibers and antioxidizing substances; and to the extemporaneous hydration of said lyophilized solid complex for reconstituting said substantially water-based vesicular dispersion just before use.

The nutriosomes of the present invention can be obtained from the association of at least: one or more phospholipid molecules, a water-soluble dextrin fiber or a mixture thereof, one or more active principles of natural origin having antioxidizing properties, dispersed together in a hydrophilic carrier prevalently or completely consisting of water or, optionally, water and water-miscible solvents, such as alcohols and/or polyalcohols, not exceeding 50% in volume, according to claim 1.

Optionally, the vesicular dispersions of the present invention may further contain at least one suitable additional polymer improving the stability of the nutriosomes and/or protecting them during the transit thereof in the acid environment of the stomach.

### Background Art

Today's unbalanced diet, poor in fermentable fibers and prebiotics, but rich in artificial sweeteners, flavoring, coloring, preservatives and other artificial additives, possibly associated with the use and abuse of alcohol, smoking and drugs, results in an alteration of the intestinal environment (dysbiosis) and an oxidative stress condition, with a final unbalance between production and elimination of highly reactive oxidizing chemical species (ROS, Reactive Oxygen Species, prevalently with a radical structure) and accumulation thereof causing cell damage, oncogenic mutations and cell death. In the long term, such alterations of the intestinal functionality lead to a number of complications that are at the basis of 60% of diseases in the modern age. Several studies have demonstrated the importance of a constant supply of dietary fibers in the treatment of intestinal dysbiosis, especially when associated with the action of antioxidizing substances avoiding the formation of free radicals or eliminating those already present. For example, almost all polyphenols of natural origin exert an antioxidizing action, and have proven to be particularly effective in the prevention and/or treatment of several pathologies due, in particular, to ROS. Merely by way of example, one of the most promising polyphenols is curcumin. Other known substances of natural origin with antioxidizing properties are, for example, ascorbic acid (or vitamin C) and/or phytocomplexes of various origin. Unfortunately, for many of these antioxidizing molecules, such as, for example, curcumin, *in vivo* efficacy is limited by their poor bioavailability and/or stability, and in order to reach the efficacious concentration on the action site it is necessary to take large quantities in repeated daily administrations.

It is known from the prior art that phospholipids dispersed in water spontaneously form double layers that close to form uni- or multi-lamellar vesicles, commonly called liposomes, which can carry both hydrophilic and lipophilic drugs. In general, liposomes are prepared with rather complex methods that include many steps and require the use of organic solvents. Moreover, these systems are used in order to carry one active agent only, very seldom two, with a variable molecular weight, and generally at low concentrations. Liposomes are very often complexed or coated with polysaccharide polymers, such as, for example chitosan, alginate, xanthan gum and derivatives of cellulose, in order to protect the phospholipids and the liposomal structure from the outside environment and modify their surface properties.

Water-soluble dextrin fibers (such as, for example, those contained in the commercial product Nutriose^{®} by Roquette, Lestrem, France) can also be loaded into liposomes to improve the distribution and deposition thereof on the mucosae of the entire digestive tract, particularly the distal tract. So far, these water-soluble dextrin fibers have been used for coating liposomes and increasing their stability in the gastrointestinal environment (see, for example, Castangia et al., 2015). In this case, however, the coating technique leads to a considerable decrease (down to half) of the concentration of the carried substance and of the liposomes themselves, because the vesicular dispersion is mixed with an equal volume of Nutriose^{®} solution. Moreover, when using this technique the usable fiber/polymer concentration is generally very low. Until now, to the authors' knowledge, water-soluble dextrin fibers have never been carried in liposomes, whether alone or together with other bioactive agents, so as to obtain a synergic, beneficial and protective action upon the intestine as a whole.

Furthermore, preserving aqueous liposome dispersions has generally proven to be problematic, especially in the presence of macromolecular components promoting liposome precipitation, aggregation and fusion.

To the present inventors' knowledge, no publications and/or patents are known which describe the preparation of complexes based on lamellar phospholipid vesicles containing water-soluble dextrin fibers and active substances of natural original with antioxidizing activity.

### Technical Problem

Therefore, a need is still felt by those skilled in the art for biocompatible and stable carriers which are capable of carrying together water-soluble fibers and natural antioxidants via oral administration, thus increasing the local and/or systemic bioavailability thereof, and which can be used for the treatment of the various forms of the gastrointestinal tract dysbiosis.

It is the object of the present invention to provide an adequate solution to the above-described technical problem.

### Brief Description of the Drawings

The present invention will now be described by way of non-limiting, example, by illustrating some preferred embodiments thereof. For this purpose, in order to better highlight the potentialities and peculiarities of the aggregated phospholipid-based nanovesicles of the present invention, reference will also be made to the annexed drawings, which present aspects and features of said nanovesicles, in which, merely by way of non-limiting example, ascorbic acid, curcumin, phycocyanin and a vinasse extract have been charged.
**Figure 1** shows some representative images, taken with a transmission electron microscope, of comparison liposomes (right), nutriosomes (center) and HPMC-nutriosomes (left) encapsulating ascorbic acid. The bars shown correspond to 100 nm.
**Figure 2** shows the values of the mean diameter (DM), polydispersity index (PI) and zeta potential (PZ) of liposomes and nutriosomes encapsulating ascorbic acid, preserved for 90 days at 25±1°C. The mean values ± standard deviation are indicated (error bars).
**Figure 3** shows the quantity of ascorbic acid released at pH 2 during 2 hours of experiment from, respectively: aqueous solution, liposomes, nutriosomes and HPMC-nutriosomes. The mean values ± standard deviation are indicated (error bars).
**Figure 4** shows the quantity of ascorbic acid released at pH 7 during 6 hours of experiment from, respectively: aqueous solution, liposomes, nutriosomes and HPMC-nutriosomes. The mean values ± standard deviation are indicated (error bars).

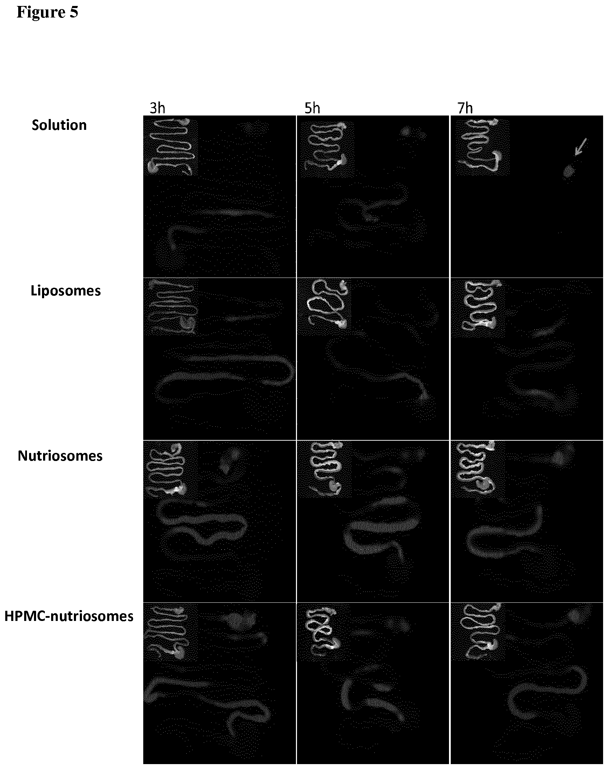

**Figure 5** shows images of the intestine of rats to which phycocyanin in aqueous solution or encapsulated into liposomes and nutriosomes was orally administered. The fluorescence of phycocyanin is shown in blue.
**Figure 6** shows some representative images, taken with a cryogenic transmission electron microscope, of comparison liposomes (A), nutriosomes (B) and HPMC-nutriosomes (C) incorporating curcumin.
**Figure 7** shows the values of the mean diameter (DM), polydispersity index (PI) and zeta potential (PZ) of liposomes and nutriosomes incorporating curcumin, lyophilized and then re-hydrated after 30, 60, 90, 120, 150 and 180 days. The mean values ± standard deviation are indicated (error bars).
**Figure 8** shows the viability of cells (Caco2) stressed with hydrogen peroxide and simultaneously treated with the aqueous dispersion of curcumin or with liposomes and nutriosomes. The mean values ± standard deviation are indicated (error bars).
**Figure 9** shows the quantity of curcumin accumulated in the different tracts of the intestine of rats to which curcumin dispersed in water or incorporated into liposomes or nutriosomes was administered by gavage. The mean values ± standard deviation are indicated (error bars).
**Figure 10** shows the plasmatic concentration of curcumin measured in rats to which curcumin dispersed in water or incorporated into liposomes or nutriosomes was administered by gavage. The mean values ± standard deviation are indicated (error bars).
**Figure 11** shows some representative images, taken with a cryogenic transmission electron microscope, of 120liposomes (A), EU-120nutriosomes (B) and EU-180nutriosomes (C) incorporating curcumin.
**Figure 12** shows the values of the mean diameter (DM), polydispersity index (PI) and zeta potential (PZ) of liposomes and EU-nutriosomes incorporating curcumin, lyophilized and then re-hydrated after 90 days. The mean values ± standard deviation are indicated (error bars).
**Figure 13** shows the viability of cells (Caco2) incubated for 48 hours with the aqueous dispersion of curcumin or with liposomes or EU-nutriosomes. The mean values ± standard deviation are indicated (error bars).
**Figure 14** shows the viability of cells (Caco2) stressed with hydrogen peroxide and simultaneously treated with the aqueous dispersion of curcumin or with liposomes and EU-nutriosomes. The mean values ± standard deviation are indicated (error bars).
**Figure 15** shows the values of the mean diameter (DM), polydispersity index (PI) and zeta potential (PZ) of liposomes and nutriosomes incorporating a vinasse extract, prepared with different concentrations of Nutriose® (20, 40, 60%) and preserved for 90 days at 25°C. The mean values ± standard deviation are indicated (error bars).
**Figure 16** shows the viability of cells (Caco2) incubated for 48 hours with an aqueous dispersion of vinasse extract or with liposomes or nutriosomes prepared with different concentrations of Nutriose^{®} (20, 40, 60%). The mean values ± standard deviation are indicated (error bars).
**Figure 17** shows the viability of cells (Caco2) stressed with hydrogen peroxide and simultaneously treated with an aqueous dispersion of vinasse extract or with liposomes or nutriosomes prepared with different concentrations of Nutriose^{®} (20, 40, 60%). The mean values ± standard deviation are indicated (error bars).

### Detailed Description of the Invention

The present invention relates to complexes formed by phospholipids, dextrin fibers and natural antioxidants that, in an aqueous environment, assemble under the form of stable lamellar vesicles according to claim 1 (hereafter referred to as *nutriosomes* for simplicity) comprising:
at least one phospholipid;
at least one water-soluble dextrin fiber;
at least one substance of natural origin having antioxidizing properties;
said vesicular complexes being dispersed in a substantially or totally water-based hydrophilic solvent.

In said vesicular complexes, the at least one phospholipid is selected from the group consisting of one or more natural or synthetic phospholipids, whether pure or as a mixture, such as, for example, soy or egg lecithin, phosphatidylcholine at different degrees of purity, whether hydrogenated or non-hydrogenated, and all derivatives thereof, phosphatidylethanolamine, phosphatidylserine, phosphatidylglycerol, phosphatidylinositol, dimyristoylphosphatidylcholine, dipalmitoylphosphatidylcholine, distearoylphosphatidylcholine, palmitoylstearoylphosphatidylcholine, sphingomyelin, mixtures of soy phospholipids, hydrogenated and non-hydrogenated, and/or mixtures of the above-mentioned phospholipids, e.g. the products commercially known as Phospholipon 90G (P90G) or Lipoid S75 (S75) (two mixtures of phospholipids prevalently containing soy phosphatidylcholine and distributed in Italy by AVG S.r.l., Garbagnate Milanese on behalf of Lipoid GmbH, Ludwigshafen, Germany); preferably, soy phosphatidylcholine, hydrogenated and non-hydrogenated, the above-described derivatives thereof, the mixtures thereof, and/or the commercial products P90G or S75 or non-purified soy lecithin commonly sold as a food supplement.

The fundamental characteristics of the phospholipids chosen for preparing the vesicular complexes of the present invention are high biologic compatibility, low metabolic degradability (i.e. *in vivo* stability) and absence of toxicity.

In said complexes, the at least one water-soluble dextrin fiber is Nutriose^{®} (a highly soluble and digestable mixture of dextrin fibers derived from wheat and maize, available from Roquette, Lestrem, France).

In said vesicular complexes, the at least one antioxidizing substance is selected from the group consisting of known natural molecules, whether pure or as a mixture, or in the form of a purified or non-purified extract, having, among any other properties, a significant antioxidizing activity, in particular against ROS. Said molecules are, for example, ascorbic acid (or vitamin C) or prevalently polyphenols, such as gallic acid, ellagic acid, resveratrol, baicalin, quercetin, curcumin; complex macromolecular substances, such as phycocyanins (photosynthetic pigments formed by a phycobiliprotein and a water-soluble pigment that gives it the characteristic blue-purple color); vegetable extracts containing different antioxidizing substances, such as grape extracts, vinasse and grape seed extracts, Citrus leaf and fruit extracts.

In said complexes, the dispersing hydrophilic solvent may consist of water only or a mixture of water-alcohols and/or water-soluble polyalcohols, wherein said alcohols are present in a quantity not exceeding 50% in volume relative to the total volume of said dispersing hydrophilic solvent.

Optionally, said vesicular complexes or nutriosomes of the present invention may further comprise an additional polymer having the function of interacting with the phospholipids and the dextrin fibers, thus rendering the formulation gastro-resistant and more stable, particularly during the transit in the acid environment of the stomach. Said additional polymer is preferably selected from the group consisting of one or more known polymers, such as, for example, cellulose derivatives, alginates, gums, copolymers of methacrylic acid and of its methyl ester (commercially known as Eudragit^{®}, Evonik Industries, Essen, Germany), and/or mixtures thereof; preferably, hydroxypropyl methylcellulose and Eudragit^{®}.

The composition of the substantially water-based dispersion of vesicular complexes of the present invention, described above, is shown in the following Table 1a).

**Table 1a): Composition of the dispersion, in aqueous carrier, of the nutriosomes of the present invention**

| **Phospholipid Component** (weight/volume %) | **Dextrin Fiber** (weight/volume %) | **Antioxidant Active** (weight/volume %) | **Additional Polymer** (weight/volume %) |
|---|---|---|---|
| **1-50** | **1-60** | **0.1 - 15** | **0 - 20** |

The quantities of the components of the nutriosomes of the present invention are expressed in component weight per 100 parts by volume of dispersion. The dispersion carrier is aqueous and formed by water (in a quantity in volume sufficient to reach 100% of the dispersion volume), or by mixtures of water and one or more water-miscible solvents suitable for oral use, such as ethanol, glycerol and propylene glycol, whether alone or as a mixture thereof, at a variable concentration of 0 to 50% (w/w).

In said composition, the phospholipid component is preferably present in a quantity ranging from 1 to 40 parts by weight per 100 parts in volume of dispersion; more preferably, from 6 to 30.

In said composition, the dextrin fiber component is preferably present in a quantity ranging from 10 to 50 parts by weight per 100 parts in volume of dispersion; more preferably, from 10 to 30.

In said composition, the antioxidizing component is preferably present in a quantity ranging from 0.1 to 10 parts by weight per 100 parts in volume of dispersion; more preferably, from 0.4 to 8.

In said composition, the additional polymeric component is preferably present in a quantity ranging from 0 to 20 parts by weight per 100 parts in volume of dispersion; more preferably, from 0 to 10.

Unlike the comparison liposomes containing the antioxidizing active principle, which are generally prepared by using complex conventional multi-stage methods involving, among other things, the use of toxic organic solvents, the dispersion of nutriosomes of the present invention is simply prepared by sonication of the mixture of the desired components, previously hydrated in the above-described substantially water-based hydrophilic solution, preferably in water.

Said method, which is also an object of the present invention, will be described in detail in the following experimental section, and essentially comprises the following steps:
- weighing all components in a suitable container, e.g. glass vials, and adding them in succession in any order (the order of addition is not restrictive); and letting them hydrate in the hydrophilic solvent, preferably water, for a time of approximately 4-8 hours, preferably 8 hours, at room temperature;
- subjecting the samples to sonication by using a conventional ultrasonic equipment commonly used in the art (such as, for example, a Soniprep 150- from MSE Crowley, UK), sonicating the mixture of components for 10-100 cycles (1-10 seconds ON, 1-10 seconds OFF) and with an amplitude of 10 to 15 µm, preferably 15 cycles (5 seconds ON, 2 seconds OFF) and with an amplitude of 13 µm, at room temperature;
to obtain the desired nanometric lamellar vesicles (the nutriosomes of the present invention) simultaneously carrying the dextrin fiber and the antioxidizing active principle.

After preparation, the water-based suspension of nutriosomes of the present invention can advantageously be subjected to lyophilization, by using standard methods and equipment as commonly used in the art, to obtain lyophilized solid complexes. In this form, the nutriosomes of the present invention are particularly stable and can be preserved, e.g. in a vacuum, without undergoing any alteration during the storage period.

Said lyophilized solid complexes can then be advantageously re-dispersed extemporaneously, just before use, into the above-mentioned hydrophilic solvent, preferably water, in order to reconstitute the vesicular dispersion of nutriosomes of the present invention, as previously described, to be used in the preparation of nutraceutical and/or pharmaceutical products intended for oral administration, for the treatment of gastro-intestinal tract disbiosis or for use as an antioxidant supplement.

As a consequence, also these lyophilized solid complexes are nutriosomes and an object of the present invention, as well as the dispersion thereof extemporaneously reconstituted before use.

The composition of the lyophilized solid complexes of nutriosomes of the present invention as previously described, expressed as percentage weight of the components with respect to the total weight of the lyophilized product, is shown in the following Table 1b).

**Table 1b): Composition of the lyophilized solid nutriosomes of the present invention**

| **Phospholipid Component** (weight %) | **Dextrin Fiber** (weight %) | **Antioxidizing Active** (weight %) | **Additional Polymer** (weight %) |
|---|---|---|---|
| **40-80** | **10-80** | **0.1 - 20** | **0-10** |

As a function of the phospholipid and dextrin fiber concentration, uni- or multi-lamellar or multi-compartmental nutriosomes can be obtained, with average sizes ranging from 50 to 500 nm; preferably from 80 to 300 nm; more preferably from 100 to 250 nm.

In the dispersion of nutriosomes of the present invention, the dextrin fiber is located both within the vesicles (in the aqueous core and in the interlamellar spaces) and in the intervesicular aqueous liquid, thus stabilizing the vesicles during the transit in the gastrointestinal tract. The dextrin fiber dissolved in the aqueous liquid exerts a dual function because, being prebiotic, it promotes intestinal health and, from a formulation viewpoint, it also behaves as a cryoprotector and causes the vesicles to maintain their structure during the lyophilization process and, after said process, to instantly re-form by extemporaneous re-hydration before use.

In comparison with conventional liposomes, in the innovative system of the present invention an antioxidizing active principle and a dextrin fiber are simultaneously carried, which exert, at gastrointestinal level, a beneficial and synergic action, rebalancing the intestinal flora and minimizing and/or preventing the negative effects and intestinal damages due to oxidative stress. Furthermore, the nutriosomes of the present invention are prepared by using a simple, eco-sustainable method not requiring the use of organic solvents, i.e. a method that can be easily reproduced at industrial level, whereas liposomes are normally prepared with complex, multi-stage methods requiring the use of organic solvents. Liposomes are generally particularly unstable in an acid environment, where they break up and release the encapsulated active principles. The nutriosomes of the present invention can be complexed or protected by means of specific polymers that render them gastro-resistant.

In addition, the creation of this type of vesicular complexes has made it possible to overcome and/or reduce the limitations encountered when using conventional liposomes and liposome-like vesicles (such as, for example, inability to incorporate large quantities of active agents, poor stability of the system in the solid state for long periods of time, and so on).

A further specific object of the present invention is therefore the use of the nutriosomes both as a substantially water-based dispersion and as a lyophilized solid in the preparation of nutraceutical and/or pharmaceutical products intended for oral administration, for use in the treatment of gastrointestinal dysbiosis or for use as antioxidant supplements.

### Experimental Section

The following experimental section will describe, by way of example, some of the characteristic aspects of the present invention, without however limiting the broad application potential thereof.

### EXAMPLE 1. Preparation of the nutriosomes according to the present invention, containing ascorbic acid as an antioxidizing agent, chemical-physical and technological characterization thereof.

Ascorbic acid is a vitamin having a low molecular weight (Vitamin C), which is largely found in foods of vegetable origin, being the principal active component of citruses. Its interest in the pharmaceutical and nutraceutical field is mainly related to its antioxidizing action, which is also exerted against ROS produced in the human organism after stresses of various types. In particular, it has been demonstrated that ascorbic acid is useful against gastrointestinal tract disorders, and particularly against the development of inflammatory states caused by excessive ROS increase. For this reason, it has been encapsulated into the nutriosomes of the present invention, which already have prebiotic properties due to the presence of Nutriose^{®}, for the purpose of improving the bioavailability of ascorbic acid at gastrointestinal tract level and promoting the correct operation of the intestine.

The composition of a preferred dispersion of nutriosomes containing ascorbic acid as an antioxidizing agent is shown in the following Table 2, in comparison with traditional liposomes containing the same quantity of ascorbic acid and with the same nutriosomes containing hydroxypropyl methylcellulose (HPMC) as an additional polymer.

**Table 2: Composition of the dispersion of nutriosomes encapsulating ascorbic acid (AA).**

| **Samples** | **P90G mg/mL** | **AA mg/mL** | **Nutriose mg/mL** | **HPMC mg/mL** | **H₂O mL** |
|---|---|---|---|---|---|
| Liposomes (control) | 160 | 10 | 0 | 0 | 1 |
| Nutriosomes | 160 | 10 | 50 | 0 | 1 |
| HPMC- Nutriosomes | 160 | 10 | 50 | 1 | 1 |

In order to prepare the nutriosomes, a commercial mixture of phospholipids obtained from soy lecithin, Phospholipon 90G (P90G), was used, mainly containing soy phosphatidylcholine (sold in Italy by AVG S.r.l., Garbagnate Milanese on behalf of Lipoid GmbH, Ludwigshafen, Germany).

The vesicles were prepared by adding to the phospholipids ascorbic acid purchased from Sigma-Aldrich (Milan, Italy) and Nutriose^{®} FM06, a commercial mixture of soluble dextrin fibers of natural origin (supplied by Roquette, Lestrem, France) and hydroxypropyl methylcellulose (HPMC), a semi-synthetic polymer derived from cellulose, purchased from Sigma-Aldrich (Milan, Italy).

The liposomes used as a comparison and encapsulating ascorbic acid were prepared by using the same method employed for the nutriosomes, i.e. by leaving the phospholipid and the ascorbic acid to hydrate for 8 hours in water, and then sonicating the dispersion.

As regards the preparation of the complexes of the present invention, all components were weighed in glass vials in succession (in any order) and left to hydrate in water for 8 hours. Subsequently, the samples were sonicated with an ultrasonic sonicator (Soniprep 150-MSE Crowley, UK) for 15 cycles (5 seconds ON, 2 seconds OFF) and with an amplitude of 13 µm.

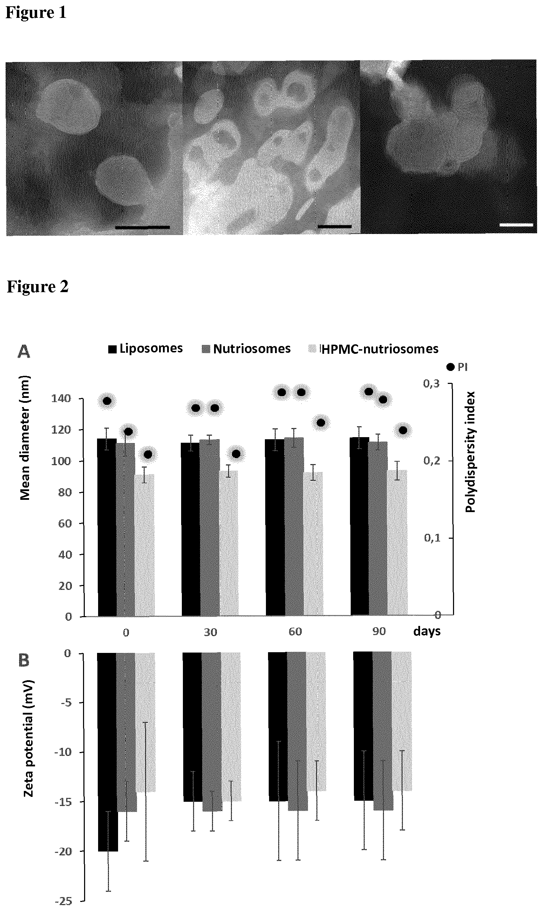

From the images obtained with a transmission electron microscope, it can be inferred that nutriosomes and HPMC-nutriosomes have a multi-lamellar structure and a spherical morphology, similar to that of the corresponding liposomes (see annexed **Figure 1**). For all vesicular dispersions, the mean diameter, the polydispersity index and the Zeta potential were measured.

**Table 3: Mean diameter (DM), polydispersity index (PI), zeta potential (PZ) and encapsulation efficiency (EE) of comparison liposomes, nutriosomes and HPMC-nutriosomes encapsulating ascorbic acid, after preparation (DP) and after lyophilization and re-hydration (DR). The mean values ± standard deviation are indicated.**

| | | **DM (nm)** | **PI** | **PZ (mV)** | **EE (%)** |
|---|---|---|---|---|---|
| Liposomes | DP | 119±8 | 0.29 | -21±4 | 61±12 |
| | DR | 174±14 | 0.37 | -18±2 | |
| Nutriosomes | DP | 124±8 | 0.26 | -10±5 | 74±11 |
| | DR | 155±4 | 0.26 | -12±3 | |
| HPMC-nutriosomes | DP | 142±4 | 0.22 | -10±3 | 73±10 |
| | DR | 163±6 | 0.23 | -13±4 | |

The nutriosomes encapsulating ascorbic acid have chemical-physical characteristics very similar to those of the comparison liposomes, prepared with the same quantity of phospholipid and ascorbic acid (Table 3). Their average size, ~133 nm, is slightly greater than that of the liposomes (∼119 nm), while the polydispersity index is slightly lower (~0.24), indicating monodisperse samples. The zeta potential of the comparison liposomes is negative (-21 mV) due to the presence of the negative charges of phosphatidylcholine, while this charge is partially neutralized in the nutriosomes of the present invention (~-11 mV), probably because of the presence of Nutriose^{®} also on the vesicular surface. The addition of the additional polymer, HPMC, does not modify the chemical-physical characteristics of the vesicles, probably because its concentration is very low compared with the other components.

The nutriosomes of the present invention are capable of encapsulating large quantities of ascorbic acid (EE% ~73%), without any statistically significant difference between the two different formulations, but the quantities are greater than those in the comparison liposomes.

After preparation (DP), the vesicles were lyophilized and then gradually re-hydrated with purified water. The size, the polydispersity index and the zeta potential measured after re-hydration (DR) are shown in Table 3. It can be observed that the size and the polydispersity index of the liposomes increase by almost 40%, whereas the size of the nutriosomes increases only by 20% and, most importantly, their polydispersity index decreases, indicating that the re-formed vesicles have very homogeneous dimensions. This confirms that Nutriose® behaves as a cryoprotector, preventing the vesicles from breaking up during the de-hydration phase and promoting their reconstitution during the re-hydration phase.

The stability of the liquid dispersions of the nutriosomes of the present invention and of the comparison liposomes was evaluated by monitoring the mean diameter, the polydispersity index and the zeta potential thereof for a period of 90 days, during which the samples were stored at 25±1 °C. Both the nutriosomes of the present invention and the liposomes have good stability; in fact, their chemical-physical characteristics remained unchanged during the period of study, indicating that no vesicle aggregation and/or fusion phenomena had occurred (see annexed **Figure 2**).

The capability of the vesicles to keep their own structure unchanged when they come in contact with solutions mimicking the different pH and ionic strength conditions encountered in the gastrointestinal tract was evaluated by diluting the dispersions with a solution having high ionic strength (NaCl 0.3 M) and maintaining them at 37°C at pH 2 for 2 hours, or at pH 7 for 6 hours. The mean diameter, polydispersity index and zeta potential of each sample were measured at the time of dilution (time zero, to), after 2 hours at pH 2 (t2h) and after 6 hours at pH 7 (t6h) (Table 4).

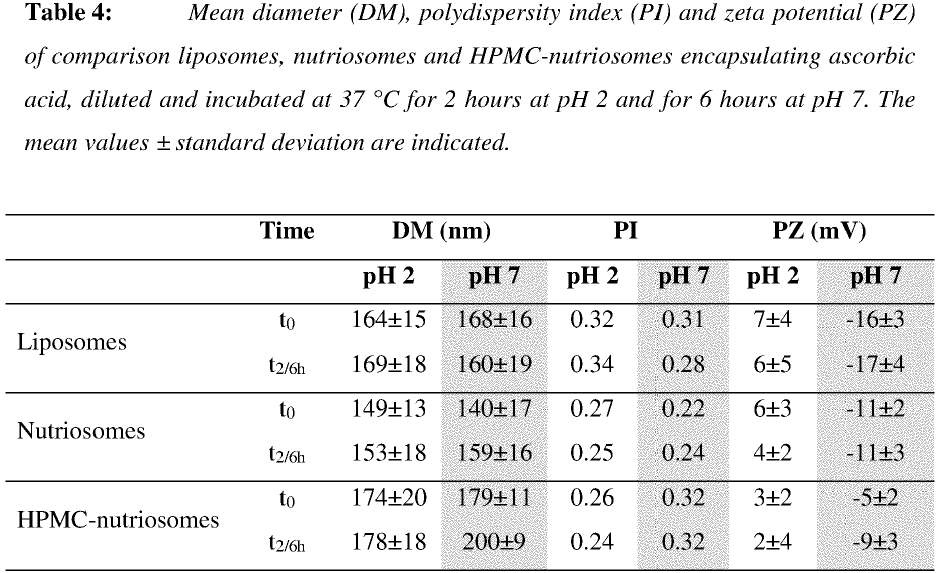

The size of the comparison liposomes increases from ~119 nm to ~165 nm in both acid and basic environments, denoting good stability, which is even better for the nutriosomes and the HPMC-nutriosomes, the dimensions of which increase very little, probably because of the presence of dextrin on the vesicular surface.

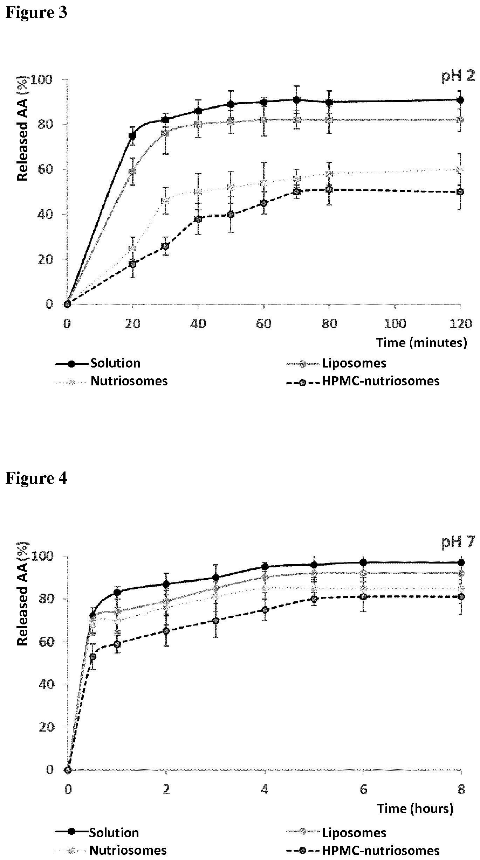

The velocity (concentration as a function of time) at which the ascorbic acid is released from the vesicles as a function of pH (2 and 7) was measured as well. The dispersions (1 mL) were transferred into dialysis tubes Spectra-Por (300 kDa Float-A-Lyzer G2) immersed in 50 mL of a solution of HCl 0.1 M (pH 2), or in 50 mL of PBS (Phosphate Buffered Saline at pH 7), and maintained at 37 °C under constant stirring for 2 hours at pH 2 and for 6 hours at pH 7. A percentage of the sample was removed at predefined time intervals, and the ascorbic acid concentration was measured with a UV spectrophotometer. The graph shows ascorbic acid concentration as a function of time (see annexed **Figure 3****)**.

As aforementioned, release studies were carried out in order to evaluate the ability of the lamellar vesicles to retain and protect the ascorbic acid during the passage through the stomach (pH 2) and/or to retain/protect the same at the neutral pH of the intestine. As can be seen in **Figure 3****,** the comparison liposomes and the aqueous solution release ascorbic acid in a very similar manner, and approximately 80% of the ascorbic acid is released after only 20 minutes. The nutriosomes of the present invention, with or without the additional polymer HPMC, release a quantity of less than 20% after 20 minutes; subsequently, the HPMC-nutriosomes release the lowest percentage, ~40%, after 120 minutes.

At pH 7, the behavior of the solution and of the liposomes is similar, since they release about 80% of the ascorbic acid after 1 hour, while the quantity released by the nutriosomes, and especially by the HPMC-nutriosomes, is smaller (~40%), as shown in the annexed **Figure 4****.** Therefore, the overall results indicate that the control liposomes do not have a significant resistance to the acid environment and release the ascorbic acid similarly to the solution, whereas the nutriosomes, and especially the HPMC-nutriosomes, are pH-sensitive, are resistant to acid pH, and release the ascorbic acid in a controlled manner at pH 7.

### EXAMPLE 2. Preparation of the nutriosomes according to the present invention encapsulating a fluorescent macromolecular probe, phycocyanin, chemical-physical characterization and evaluation of the in vivo biodistribution thereof

The same nutriosome formulations as presented in Example 1 were used for encapsulating phycocyanin, a water-soluble macromolecule belonging to the family of phycobiliproteins. Phycocyanin is one of the main constituents of red and blue algae; it is formed by a proteic unit and a chromophore pigment that makes it fluorescent. In this study, phycocyanin was used as a fluorescent probe in order to evaluate the *in vivo* destiny of the hydrophilic macromolecules carried in the nutriosomes. Therefore, its intestinal biodistribution gives an indication as to the possible destiny of the dextrin fiber (Nutriose®), which is otherwise very difficult to detect *in vivo* after oral administration.

The composition of an example of dispersion of the complexes of the present invention (nutriosomes), containing phycocyanin, is show in the following Table 5, which also indicates the composition of the comparison liposomes.

**Table 5: Composition of the dispersion of nutriosomes containing phycocyanin.**

| | **P90G (mg/mL)** | **Phycocyanin (mg/mL)** | **Nutriose (mg/mL)** | **HPMC (mg/mL)** |
|---|---|---|---|---|
| Liposomes | 160 | 20 | 50 | 0 |
| Nutriosomes | 160 | 20 | 50 | 0 |
| HPMC-nutriosomes | 160 | 20 | 50 | 1 |

The formulations of the nutriosomes of the present invention containing phycocyanin were prepared by following the method previously described for the nutriosomes encapsulating ascorbic acid. For preparation, phycocyanin was isolated from AfaMax^{®}, an extract of AFA-phycocyanins from Klamath algae, supplied by Nutratec (Urbino, Italy). Based on the method described by Benedetti *et al.* 2006 (Benedetti *et al.,* 2006), some modifications as described by Caddeo *et al.* 2013 (Caddeo *et al.,* 2013) were made. In brief, AfaMax (500 mg) was suspended in 50 mL of PBS, and ammonium sulfate was added in order to cause the phycocyanin to precipitate (salting-out effect). The suspension was then centrifuged, the colorless supernatant removed, and the blue precipitate dissolved into a small volume of PBS and dialyzed overnight at 4 °C in PBS. The dialyzed phycocyanin was then purified by gel filtration chromatography by using, as a stationary phase, Sepharose^{®} CL-4B (Sigma-Aldrich, Milan, Italy); the eluate was lyophilized, thereby obtaining a blue powder characterized, from a qualitative and quantitative viewpoint, as described by Caddeo *et al.,* 2013. After preparation, the liposomes and nutriosomes were lyophilized and then gradually re-hydrated with purified water. The size, polydispersity index and zeta potential measured before and after lyophilization are shown in Table 6.

**Table 6: Mean diameter (DM), polydispersity index (PI) and Zeta potential (PZ) of liposomes and nutriosomes encapsulating phycocyanin after preparation (DP) and after lyophilization and re-hydration (DR). The mean values ± standard deviation are indicated.**

| | **DM (nm)** | **PI** | **PZ (mV)** |
|---|---|---|---|
| Liposomes (DP) | 105±3 | 0.35 | -14±1 |
| Liposomes (DR) | 132±5 | 0.33 | -17±1 |
| Nutriosomes (DP) | 151±1 | 0.34 | -15±1 |
| Nutriosomes (DR) | 166±4 | 0.33 | -16±1 |
| HPMC-nutriosomes (DP) | 163±3 | 0.32 | -15±1 |
| HPMC-nutriosomes (DR) | 170±6 | 0.31 | -16±1 |

After preparation, the nutriosomes of the present invention showed average dimensions of ~155 nm, i.e. slightly greater than those of the liposomes, which were ~100 nm. After lyophilization and re-hydration, the nutriosomes maintain the same dimensions, whereas those of the comparison liposomes increase slightly (Table 6). The Zeta potential of the nutriosomes of the present invention is the same as that of the comparison liposomes (~ -15 mV).

The biodistribution at gastrointestinal level of the hydrophilic molecule in solution or carried in liposomes and nutriosomes was evaluated *in vivo,* by using male rats WISTAR (8-12 weeks of age, 230-250 g). The animals were treated in accordance with the European Union regulations concerning the use of laboratory animals. The protocols had been approved by the Institutional Animal Care and Use Committee of the University of Valencia (Spain). Throughout the study, the animals were kept in a thermally conditioned chamber, with a light/dark cycle of 12 hours and free access to water and food. Prior to each experiment, they were kept without food for 12 hours in order to keep the gastrointestinal tract clean. The different formulations (2 mL) were administered by gavage by using a probe. The rats were sacrificed 3, 5 and 7 hours after administration, the gastrointestinal apparatus was removed, and the distribution of phycocyanin (blue fluorescence) was observed by using an *In Vivo Imaging System FX PRO* (Bruker Bio-Spin, U.S.A.).

In the intestine of the rats treated with the aqueous solution of phycocyanin, a slight fluorescence can be observed in the distal tract after 3 hours from administration, which disappears completely after 7 hours, indicating the complete elimination of the hydrophilic macromolecule. In the intestine treated with liposomes, after 3 hours the fluorescence has a greater extension and remains, even though the signal is weak, in the whole intestine for up to 7 hours. In the intestine treated with the nutriosomes of the present invention, after 3 hours fluorescence is visible in both the stomach and the distal tract of the intestine and, although weak, reaches the colon. After 5 hours, fluorescence is particularly evident in the jejunum and in the tract immediately preceding the caecum, and so is after 7 hours, even though its intensity decreases. In the intestine treated with HPMC-nutriosomes, the fluorescence has a greater distribution, after 3 hours it is very evident in the stomach and in the jejunum, reaching also the caecum and the colon, after 5 and 7 hours it is evident especially in the stomach and in the distal tract preceding the caecum, while in the caecum and in the colon fluorescence is weak. The results indicate that the nutriosomes, unlike the reference solution and the liposomes, allow the hydrophilic macromolecule (and hence, presumably, also the hydrophilic dextrin fiber) to initially (3 hours) accumulate in larger amounts on all the gastrointestinal mucosa of the stomach down to the caecum, and then (5-7 hours) especially in the jejunum, while in the caecum most of the pigment is degraded. HPMC-nutriosomes increase the accumulation of the macromolecule, especially after 3 hours, in the caecum and the colon.

### EXAMPLE 3. Preparation of the nutriosomes according to the present invention incorporating curcumin as an antioxidizing active principle, chemical-physical characterization and evaluation of the in vitro and in vivo efficacy thereof

Curcumin (or 1,7-Bis(4-hydroxy-3-methoxyphenyl)hepta-1,6-diene-3,5-dione; Sigma-Aldrich, Milan, Italy), is a phenolic substance of natural origin found in the rhizome of *Curcuma longa.* It is a strongly hydrophobic yellow-orange powder, and it has been known and used for a long time for the treatment of several disorders of the respiratory tract (such as, for example, asthma, bronchitis, allergies), for the treatment of hepatic disorders, and for use as anti-inflammatory and antioxidant (like most polyphenols).

Curcumin has been incorporated into the nutriosomes of the present invention; a preferred composition is shown in the following Table 7 along with that of comparison liposomes.

**Table 7: Composition of the aqueous dispersion of nutriosomes containing curcumin.**

| | **P90G (mg/mL)** | **Curcumin (mg/mL)** | **Nutriose (mg/mL)** | **HPMC (mg/mL)** |
|---|---|---|---|---|
| Liposomes | 160 | 10 | 0 | 0 |
| Nutriosomes | 160 | 10 | 50 | 0 |
| HPMC-nutriosomes | 160 | 10 | 50 | 1 |

In order to prepare the nutriosomes of the present invention incorporating curcumin, all components were weighed in glass vials in succession (the addition order is not restrictive) and left to hydrate in water for 8 hours. The samples were subsequently sonicated by using an ultrasonic sonicator apparatus *(Soniprep 150-MSE* Crowley, UK), sonicating the samples for 15 cycles of 5 seconds with pauses of 2 seconds, at least 3 times, with an amplitude of 13 µm. The vesicular dispersions thus obtained were then characterized in terms of size, polydispersity index and zeta potential, before and after the lyophilization process. In order to evaluate the vesicles' ability to retain curcumin and protect it during the gastrointestinal transit, the stability of the formulations as a function of pH (2 and 7) was studied. The antioxidizing power of curcumin in dispersion or incorporated into liposomes and nutriosomes was measured *in vitro* by using Caco2 cells. The latter are a cell line isolated from human colorectal adenocarcinoma, which differentiate into a monolayer of polarized cells, coupled by junctions, which express many morphofunctional characteristics of the absorbent epithelium of the small intestine *(ATCC collection,* Sesto San Giovanni, Milan, Italy). The plasmatic concentration and the intestinal accumulation of curcumin in rats were also measured *in vivo* after gavage administration.

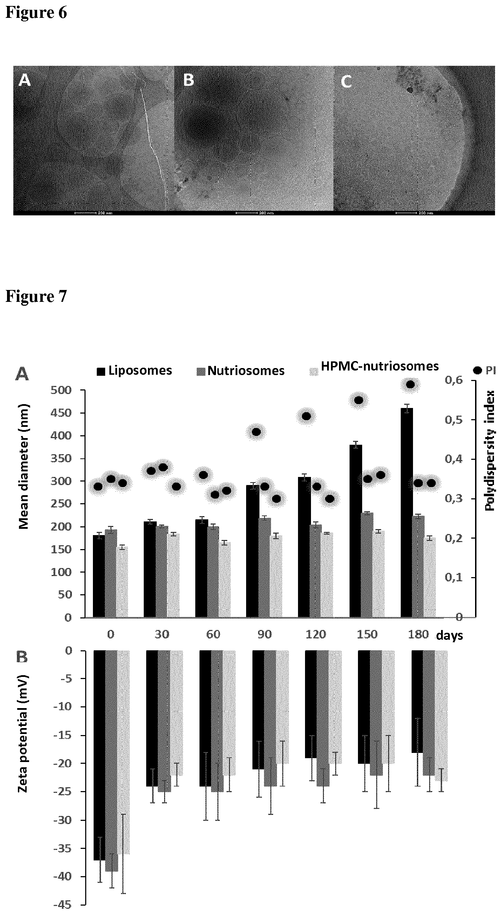

As shown in the previous examples, the complexes of the present invention form closed lamellar structures in the form of vesicles. In fact, the images obtained with the cryogenic transmission electron microscope highlight that nutriosomes and HPMC-nutriosomes are spherical and prevalently uni-lamellar, with morphological and structural characteristics that are very similar to those of the reference liposomes **(****Figure 6**). The chemical-physical characteristics of the reference liposomes and of the nutriosomes of the present invention, as well as their incorporation efficiency, are shown in Table 8.

**Table 8: Mean diameter (DM), polydispersity index (PI), Zeta potential (PZ) and incorporation efficiency (EE) of comparison liposomes, nutriosomes and HPMC-nutriosomes incorporating curcumin. The mean values ± standard deviation are indicated.**

| | **DM (nm±DS)** | **PI** | **PZ (mV±DS)** | **EE (% ±DS)** |
|---|---|---|---|---|
| Liposomes | 181±7 | 0.33 | -37±4 | 73±6 |
| Nutriosomes | 183±8 | 0.33 | -39±2 | 88±7 |
| HPMC-nutriosomes | 155±5 | 0.34 | -36±7 | 91±6 |

The nutriosomes of the present invention have an average size of ~193 nm, with a polydispersity index of ~0.33 like the comparison liposomes, whereas those containing also HPMC have slightly smaller dimensions. The incorporation efficiency of both nutriosomes (~90%) is higher than that of the liposomes (~73%), confirming the fact that the dextrin-based structure promotes the incorporation of curcumin into the network of the complex.

In order to evaluate the preservability of the dispersions, the vesicles were lyophilized, preserved in a vacuum, away from light, at 25 °C, and then extemporaneously re-hydrated with water at predefined time intervals. The characteristics of the vesicles (size, polydispersity index, zeta potential) after re-hydration are shown in the graphs of **Figure** 7.60 days later, the re-hydrated liposomes have dimensions comparable with the initial ones, but after 90 days the dimensions of the reconstituted liposomes and the polydispersity index increase to ~450 nm and ~0.60, respectively, which values indicate an unstable and very polydisperse system. On the contrary, the nutriosomes and the HPMC-nutriosomes retain the same dimensions and the same polydispersity index throughout the length of the stability study, probably because Nutriose® behaves as a cryoprotector and prevents the vesicles, when dehydrated, from breaking up, so that, when extemporaneously re-hydrated, they will re-form with the same dimensions, even after 180 days of preservation.

In order to evaluate the stability of the vesicles in the gastrointestinal environment, the formulations were diluted with two different solutions mimicking *in vitro* two of the most destabilizing factors that are encountered *in vivo* during the transit in the digestive apparatus: pH and ionic strength. Each sample was diluted with a solution at pH 2 with high ionic strength (NaCl 0.3 M) and kept under constant stirring for 2 hours at 37 °C, or diluted with a solution at pH 7 with high ionic strength (NaCl 0.3 M) and kept under constant stirring for 6 hours at 37 °C. The mean diameter, polydispersity index and zeta potential of each sample were measured at predefined time intervals, as shown in the following Table 9.

**Table 9: Mean diameter (DM), polydispersity index (PI), zeta potential (PZ) and incorporation efficiency (EE) of liposomes and nutriosomes incorporating curcumin diluted with a solution at pH 2 and a solution at pH 7 and kept at 37°C for 2 or 6 hours.**

| | **pH** | **Time** | **DM (nm)** | **PI** | **PZ (mV)** | **EE (%)** |
|---|---|---|---|---|---|---|
| Liposomes | 2 | t₀ | 205±40 | 0.41 | 13±3 | |
| | | t₂ₕ | 557±20 | 0.58 | 11±2 | 79±6 |
| Nutriosomes | 2 | t₀ | 174±15 | 0.37 | 13±4 | |
| | | t₂ₕ | 394±49 | 0.50 | 8±5 | 93±10 |
| HPMC-nutriosomes | 2 | t₀ | 199±19 | 0.40 | 10±4 | |
| | | t₂ₕ | 224±42 | 0.49 | 9±5 | 89±9 |
| Liposomes | 7 | t₀ | 181±20 | 0.42 | 0±2 | |
| | | t₆ₕ | 162±35 | 0.39 | -1±2 | 33±12 |
| Nutriosomes | 7 | t₀ | 170±7 | 0.47 | 0±2 | |
| | | t₆ₕ | 136±49 | 0.41 | -4±3 | 49±5 |
| HPMC-nutriosomes | 7 | t₀ | 134±21 | 0.48 | -2±5 | |
| | | t₆ₕ | 204±18 | 0.49 | -1±2 | 56±7 |

At pH 2, the dimensions of the liposomes undergo an important increase from ~181 to ~557 nm, and also the polydispersity index increases up to ~0.58, indicating a very heterogeneous population with vesicles having very different sizes. The quantity of curcumin released during the 2 hours is ~20% of the initial quantity. Therefore, the liposomes' dimensions tend to grow through the effect of the acid pH, but the vesicles are not destroyed and retain a large part of the curcumin within their structure. This good resistance to acid pH and ionic strength can be explained by the high concentration of the phospholipid that is partially hydrolyzed in an acid environment, while the residual quantity of phospholipid allows the vesicular structure to remain unaffected. The nutriosomes' dimensions grow less than those of the comparison liposomes, from ~183 to ~394 nm, and so does the polydispersity index, while the quantity of curcumin released decreases to ~7%, indicating that the addition of Nutriose^{®} contributes to increasing the resistance of the vesicles in the acid environment. The higher resistance of the vesicles in an acid environment is obtained by adding the additional polymer HPMC, which interacts with Nutriose^{®}. In fact, the vesicles' dimensions after 2 hours at pH 2 at 37 °C are ~224 nm, i.e. very similar to those of the initial vesicles; the polydispersity index is <0.5 and the quantity of curcumin released is ~10% of the initial one. It can therefore be stated that HPMC-nutriosomes are stable in these conditions and retain their initial chemical-physical and technological characteristics.

After 6 hours at pH 7, the dimensions of the nutriosomes of the present invention, just like those of the corresponding liposomes, do not vary significantly, and this indicates a good resistance of the vesicles to intestinal conditions. The quantity of curcumin lost during the 6 hours of study is greater for liposomes (~67%) and smaller for both nutriosomes (~47%), which are therefore able to control curcumin release.

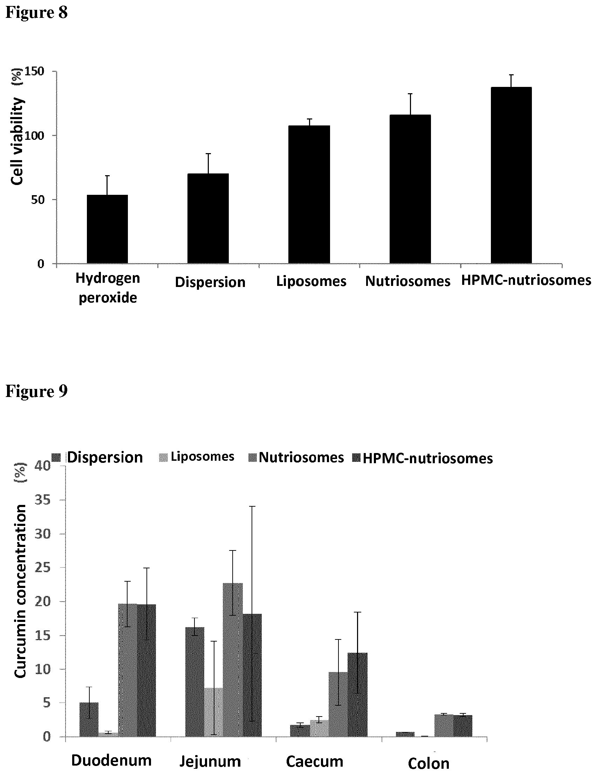

A study was carried out to evaluate the *in vitro* ability of the nutriosomes to improve the protective efficacy of curcumin against the oxidative damage induced in Caco2 cells by hydrogen peroxide **(****Figure 8**). The cells were sown in 96-well plates and incubated at 37 °C in an atmosphere with 5% CO₂ for the period necessary for achieving confluence. As a negative control, cells maintained in optimal growth conditions and not treated either with hydrogen peroxide or with curcumin were used; as a positive control, cells maintained in optimal growth conditions and treated with hydrogen peroxide, but not treated with curcumin, were used. The other cells were treated with hydrogen peroxide and, simultaneously, with curcumin in aqueous dispersion or carried in liposomes, nutriosomes and HPMC-nutriosomes. The cells were incubated for 4 hours, washed with PBS, and then cell survival was measured by means of a colorimetric test with tetrazolium salt, 3-(4,5-dimethylthiazol-2-yl)-2,5-diphenyltetrazolium bromide (MTT) (see annexed **Figure 8****)**. Cell viability was calculated as a percentage of the viability of the untreated cells (negative control).

The viability of the cells treated with hydrogen peroxide (positive control) is ~50%; the addition of curcumin in aqueous dispersion increases cell survival (~69%), and carrying curcumin in liposomes and nutriosomes leads to a further increase, up to ~110%, and to as much as ~136% with HPMC-nutriosomes, which are therefore capable of enhancing the efficacy of the polyphenol in cell cultures.

Curcumin in dispersion or carried in liposomes or nutriosomes was administered to rats *in vivo,* and both the local accumulation and the plasmatic concentration were measured. The experiments were conducted by using WISTAR male rats (8-12 weeks of age and 230-250 g). The animals were treated in accordance with the European Union regulations concerning the use of laboratory animals. The protocols have been approved by the Istitutional Animal Care and Use Committee of the University of Valencia (Spain). Throughout the length of the study, the animals were kept in a thermally conditioned chamber, with a light/dark cycle of 12 hours and free access to water and food. Prior to each experiment, they were kept without food for 12 hours in order to keep the gastrointestinal tract clean. The different formulations (2 mL) were administered by gavage to healthy rats by using a probe. In order to measure the plasmatic concentration, a cannula was inserted into the jugular vein and blood samples were taken at predefined time intervals. In order to measure the quantity of curcumin accumulated in the intestine, the rats were sacrificed 4 hours after administration, and the gastrointestinal apparatus was removed and divided into the different segments. The results are shown in the annexed **Figure 9**.

In the animals treated with the aqueous dispersion and with the liposomes, curcumin distribution is similar, and the curcumin accumulates mainly in the jejunum, without however reaching the caecum and the colon. Administration of curcumin incorporated into nutriosomes and HPMC-nutriosomes according to the present invention promotes accumulation in the different tracts of the intestine; in particular, accumulation is higher in the duodenum and in the jejunum (~120%) and lower, but still high, in the caecum (~10%) and in the colon (∼5%). Therefore, the study confirms the ability of the nutriosomes and HPMC-nutriosomes to promote local accumulation of curcumin in the whole intestinal tract.

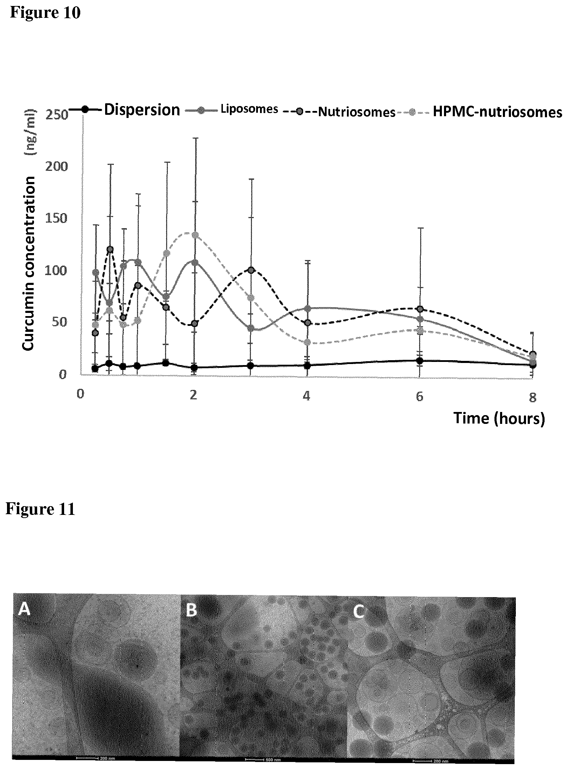

In order to quantify a plasmatic concentration of curcumin when administering the aqueous dispersion, it was necessary to double the concentration; therefore, 2 mL of dispersion were administered, containing 20 mg/mL of curcumin instead of 10 mg/mL as in liposomes and nutriosomes **(****Figure 10**). Notwithstanding this, the measured plasmatic concentration is always very low, i.e. ∼10 ng/mL during the 8 hours of study. The plasmatic profile of the curcumin administered in the different vesicular formulations (liposomes, nutriosomes and HPMC-nutriosomes) is very similar; the average plasmatic concentration is ∼80 ng/mL, but it is generally very fluctuating, with peaks at ~130 ng/mL and lows at ∼50 ng/mL: this trend is probably due to a rapid entero-hepatic circle.

Administration of curcumin and dextrin fiber carried in the dispersions of nutriosomes according to the present invention promotes, therefore, both a more uniform distribution of the polyphenol throughout the intestinal tract, better than the one given by the comparison liposomes, and the achievement of a plasmatic concentration of curcumin that is comparable to the one obtained with liposomes. Moreover, as demonstrated by the study conducted by using phycocyanin, the nutriosomes, and especially the HPMC-nutriosomes, allow for greater local accumulation of hydrophilic molecules (and therefore also of dextrins) on the whole intestinal tract, including the caecum and the colon.

### EXAMPLE 4. Preparation of nutriosomes according to the present invention incorporating curcumin and modified with the addition of a gastro-resistant polymer, chemical-physical characterization and in vitro evaluation of the stability in the intestinal environment and of the antioxidizing efficacy thereof

Curcumin was incorporated into the nutriosomes of the present invention, which were prepared by using the phospholipid Lipoid S75, prevalently containing soy phosphatidylcholine (supplied by AVG S.r.l., Garbagnate Milanese, Italy, on behalf of Lipoid GmbH, Ludwigshafen, Germany) and Nutriose^{®} FM06 by Roquette (Lestrem, France). Compared with the previous formulations, the phospholipid was used at two different concentrations (120 and 180 mg/mL), Nutriose^{®} was used at a higher concentration (100 mg/mL), and a gastro-resistant polymer, Eudragit L100 (Evonik Industries, Essen, Germany) was added. A preferred composition is shown in the following Table 10, along with the one of the comparison liposomes. This study evaluated the effect of the addition of Eudragit L100 (EU-nutriosomes) and of the lipid concentration on the stability and efficacy of the *in vitro* formulations. As a comparison, liposomes containing 120 mg/mL and 180 mg/mL of S75 were prepared, as in the corresponding nutriosomes. Unfortunately, the former have a size of ~700 nm and are unstable; in fact, curcumin precipitates after a short time. For this reason, the study was conducted by using only the liposomes containing 180 mg/mL of S75.

**Table 10: Composition of the aqueous dispersion of nutriosomes containing Eudragit L100 and incorporating curcumin.**

| | **S75 (mg/mL)** | **Curcumin (mg/mL)** | **Nutriose (mg/mL)** | **Eudragit (mg/mL)** |
|---|---|---|---|---|
| 180liposomes | 180 | 10 | 0 | 0 |
| EU-120nutriosomes | 120 | 10 | 100 | 100 |
| EU-180nutriosomes | 180 | 10 | 100 | 100 |

In order to prepare the EU-nutriosomes, curcumin, Nutriose^{®} FM06 and Eudragit L100 were weighed in a glass vial in succession (the order of addition is not restrictive); water was then added, and the dispersion was sonicated for 25 cycles of 3 seconds with pauses of 5 seconds; the phospholipid was then added, which was left to hydrate for 8 hours. The dispersions thus obtained were sonicated by using an ultrasonic sonicator apparatus (*Soniprep 150-MSE* Crowley, UK) for 50 cycles of 5 seconds with pauses of 2 seconds, with an amplitude of 13 µm.

The vesicles were observed under a cryogenic transmission electron microscope, and some representative images are shown in **Figure 11**. The vesicles are spherical, oligo-lamellar, and many of them are multi-compartmental (multi-vesicular). This phenomenon is probably due to the high concentrations of the three components. As shown in Table 11, the values of the dimensions of the comparison liposomes are high (~373 nm), and so is the polydispersity index (~0.45), whereas the addition of Nutriose^{®} and Eudragit promotes the packing of the vesicles with a shorter radius of curvature, smaller dimensions (∼300 nm), and a reduced polydispersity index (~0.20), which indicates a monodisperse sample.

**Table 11: Mean diameter (DM), polydispersity index (PI), Zeta potential (PZ) and incorporation efficiency (EE) of comparison liposomes, EU-120nutriosomes and EU-180nutriosomes.**

| | **DM (nm)** | **PI** | **PZ (mV)** | **EE (%)** |
|---|---|---|---|---|
| 180liposomes | 372±49 | 0.45 | -75±9 | 83±12 |
| EU-120nutriosomes | 299±16 | 0.18 | -71±5 | 86±6 |
| EU-180nutriosomes | 337±37 | 0.22 | -74±4 | 79±6 |

The zeta potential of the vesicles is strongly negative because of the negative charges of the lipids contained in S75.

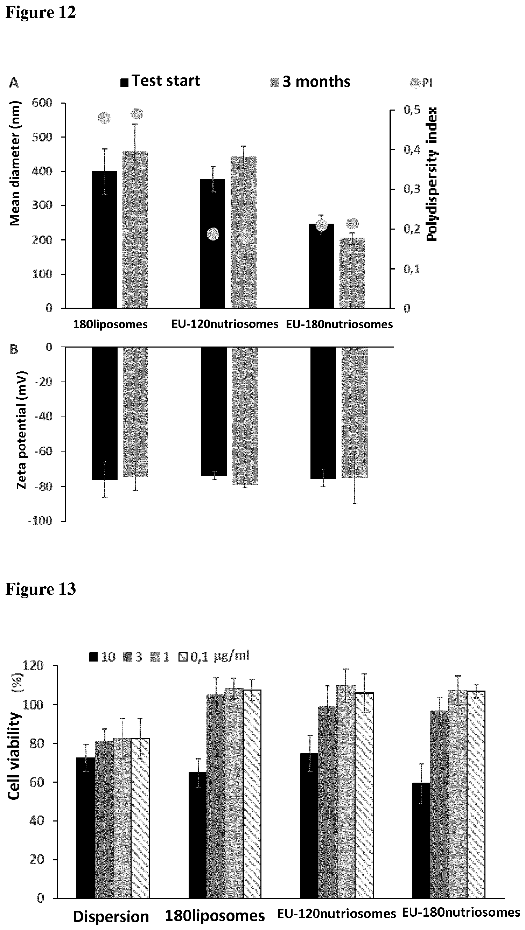

The vesicles were lyophilized and preserved for 90 days, away from light and air, at 25°C, and then extemporaneously re-hydrated with bi-distilled water. The chemical-physical characteristics of the lyophilized and re-hydrated vesicles are shown in the graphs of the annexed **Figure 12****.** The dimensions of the liposomes and their polydispersity index grow slightly after re-hydration, also as a function of time. The dimensions of the EU-120nutriosomes increase after 90 days (~441 nm), while the polydispersity index remains low (~0.18). The dimensions of the EU-180nutriosomes become smaller after lyophilization and re-hydration, and the polydispersity index stays at 0.20, indicating the existence of a monodisperse system. The zeta potential values do not change after the lyophilization and re-hydration process. Since the gastro-resistant polymer was added to the formulation, also for the EU-nutriosomes we tested the stability at pH 2 and 7 in the presence of a high ionic strength, as shown in the previous example. The results are summarized in the following Table 12.

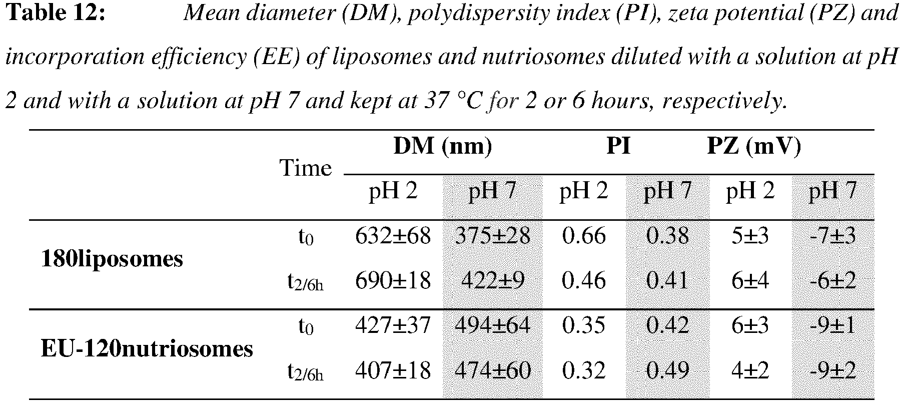

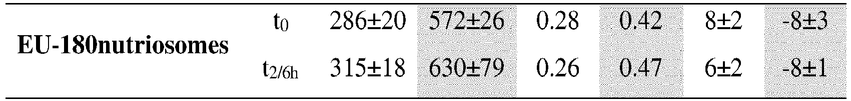

The size of the liposomes in acid environment becomes almost twice the initial value immediately after dilution, with a very high polydispersity index indicating the presence of very big particles. EU-120nutriosomes increase in size by about 100 nm, while the size and the polydispersity index of EU-180nutriosomes remain constant, confirming their ability to remain unaltered in these conditions thanks to the simultaneous presence of Eudragit and of the high concentration of S75. In these conditions, the zeta potential reverses and becomes positive. At pH 7, the dimensions of the liposomes and EU-120nutriosomes increase slightly, whereas those of the EU-180nutriosomes double, probably because the vesicles begin breaking up and a large part of the curcumin is released.

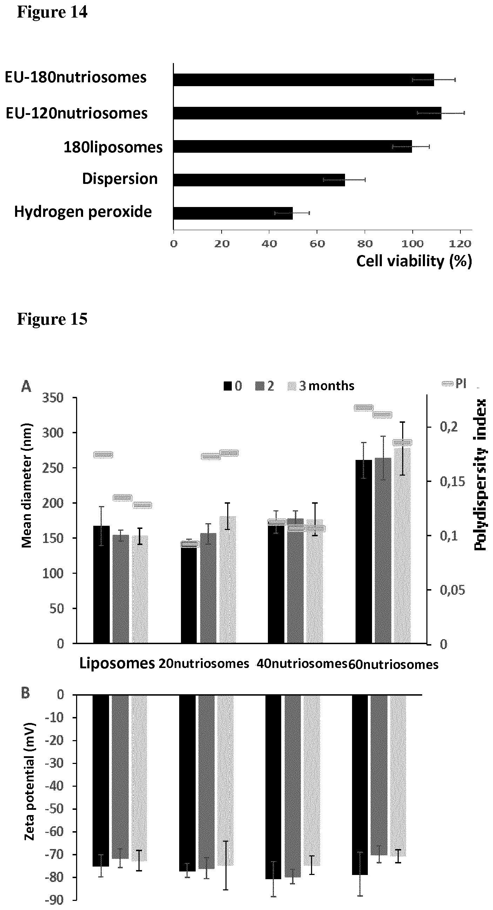

The biocompatibility of curcumin in dispersion or carried in liposomes or EU-nutriosomes was measured by treating intestinal cells (Caco2) with the vesicular formulations at different dilutions for 48 hours. At the end of this incubation period, cell viability was measured by MTT test, and was expressed as a percentage with respect to the untreated cells (negative control). The results are shown in the annexed **Figure** 13. At the lowest dilution (10 µg/mL of curcumin), viability is ~75%, regardless of the tested sample; at higher dilutions, the viability of the cells treated with curcumin in dispersion remains around 80%, while that of the cells treated with the nutriosomes is greater (~105%) and equal to that of the reference liposomes, which are generally known to be a non-toxic and biocompatible carrier. Probably all vesicular systems are capable of modulating the curcumin uptake within the cells, thereby reducing any toxic effect thereof. By using a non-toxic dilution (1 µg/mL of curcumin), we studied the ability of the formulations to promote the antioxidizing efficacy of the polyphenol. As shown in the previous example, the cells were stressed with hydrogen peroxide and simultaneously treated with curcumin in dispersion or incorporated in the vesicles (see annexed **Figure 14**). In this case as well, the cells stressed with hydrogen peroxide have a viability of ~50%, and the addition of the dispersion increases viability up to ~70%. The viability of the cells treated with the liposomes is ~100%, and that of the EU-nutriosomes reaches ∼110%, confirming the very good properties as carriers of these vesicular systems.

### EXAMPLE 5. Preparation of nutriosomes according to the present invention incorporating vinasse extract and obtained with increasing concentrations of dextrins, chemical-physical characterization and in vitro evaluation of the stability in the intestinal environment and of the antioxidizing efficacy thereof

Grape extract was obtained by extraction from vinasse of cannonau grapes in ethanol and propylene glycol (1:1 v/v) and subsequent dilution and concentration of the extract by lyophilization (Manca *et al.,* 2016; Manconi *et al.,* 2016). The fluidic extract thus obtained was incorporated into the nutriosomes of the present invention, which were prepared by using the phospholipid Lipoid S75 (supplied by AVG S.r.l., Garbagnate Milanese, Italy, on behalf of Lipoid GmbH, Ludwigshafen, Germany) and increasing quantities of Nutriose^{®} FM06 by Roquette (Lestrem, France). A preferred composition is shown in the following Table 13, along with the one of the comparison liposomes.

**Table 13: Composition of nutriosomes containing increasing quantities of Nutriose^{®} and incorporating vinasse extract.**

| | **S75 (mg/mL)** | **Nutriose (mg/mL)** | **Extract (mg/mL)** |
|---|---|---|---|
| Liposomes | 60 | 0 | 45 |
| 20nutriosomes | 60 | 200 | 45 |
| 40nutriosomes | 60 | 400 | 45 |
| 60nutriosomes | 60 | 600 | 45 |

In order to prepare the nutriosomes, the phospholipid, Nutriose^{®} FM06 and the extract were weighed in a glass vial; water was then added, and the dispersion was left to hydrate for 8 hours, and then sonicated for 40 cycles of 5 seconds with pauses of 3 seconds, by using an ultrasonic sonicator apparatus (*Soniprep 150-MSE* Crowley, UK).

The liposomes have a size of ~167 nm, are monodisperse (polydispersity index ~0.17), have a strongly negative zeta potential (∼-75 mV) and high incorporation efficiency, as shown in Table 14. The addition of Nutriose^{®} at a low concentration (20%) allows achieving good packing of the smaller vesicles (~144 nm) and a very low polydispersity index (~0.09), indicating a high homogeneity of the system. The zeta potential and the incorporation efficiency are comparable to those of the liposomes.

**Table 14: Mean diameter (DM), polydispersity index (PI), zeta potential (PZ) and incorporation efficiency (EE) of liposomes and nutriosomes incorporating vinasse extract.**

| | **DM (nm)** | **PI** | **PZ (mV)** | **EE (%)** |
|---|---|---|---|---|
| Liposomes | 167±27 | 0.17 | -75±5 | 98±6 |
| 20nutriosomes | 144±6 | 0.09 | -77±4 | 95±7 |
| 40nutriosomes | 173±15 | 0.11 | -81±8 | 87±11 |
| 60nutriosomes | 261±26 | 0.17 | -79±9 | 85±13 |

The addition of larger quantities of Nutriose^{®} leads to a linear increase in the size of the vesicles, which, with 60% of dextrin, reaches 261 nm, while the other parameters remain unchanged.

The vesicular dispersions were preserved in liquid form for 90 days at 5 °C. The values of the mean diameter, polydispersity index and zeta potential are shown in the graphs of the annexed **Figure** 15. The dimensions of all vesicles, the polydispersity index and the zeta potential remained constant throughout the preservation period, confirming the good stability of the vesicles, also in the presence of high concentrations of Nutriose^{®}.

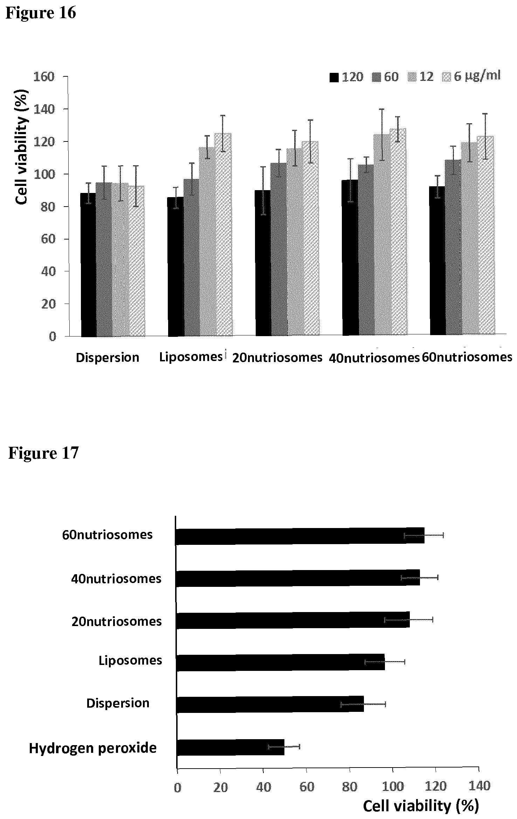

The biocompatibility of the vinasse extract in dispersion or carried in liposomes or nutriosomes was measured by treating intestinal cells (Caco2) with the formulations at different dilutions and leaving them under incubation for 48 hours. After 48 hours, cell viability was measured by MTT test and was expressed as a percentage with respect to the untreated cells (negative control). The results are shown in the annexed **Figure 15**. At the lowest dilution (120 µg/mL of extract) of both the dispersion and the vesicles, viability is (~90%); at higher dilutions, the viability of the cells treated with the extract in dispersion does not change (~95%), while that of the cells treated with nutriosomes increases (∼105%) with the next dilution (60 µg/mL of extract) and reaches ∼120% with the two highest dilutions. It appears, therefore, that nutriosomes, just like liposomes, are capable of modulating the uptake of the extract within the cells, thereby reducing the toxicity thereof. By using the non-toxic dilution of 12 µg/mL of extract, we measured the ability of the formulations to promote the antioxidizing efficacy of the extract. The cells were stressed with hydrogen peroxide (viability ~50%) and simultaneously treated with the extract in dispersion or incorporated into liposomes or nutriosomes (see annexed **Figure 17**). The addition of the dispersion to the stressed cells increases viability to ~85%, the addition of the liposomes increases it to ~96%, and the addition of the nutriosomes (regardless of the Nutriose^{®} concentration) leads to a viability in excess of ∼113%, confirming the very good properties as carriers of these vesicles.

### Advantages/Industrial Applicability of the Invention

The complexes of the present invention allowed to attain the following advantages:
- increased stability compared with conventional liposome-based systems, which tend over time to aggregate and fuse and release the carried active substances, especially because they are systems in aqueous dispersion; on the contrary, the nutriosomes of the present invention can be lyophilized and preserved in solid form for very long periods of time;
- increased ability to carry a larger quantity of lipophilic active principles, thanks to the presence of dextrins, which are to be found both within the vesicle (in the aqueous core) and within the intervesicular space;
- increased resistance to the acid environment of the stomach, especially because of the addition of a polymer that interacts with dextrins and phospholipids;
- increased antioxidizing efficacy upon the intestinal tissues, due to the function of the carrier;
- increased *in vivo* intestinal distribution and accumulation of both the dextrin and the antioxidizing active substances, resulting in greater efficacy in the treatment of intestinal dysbiosis;
- increased systemic absorption compared with the aqueous dispersion, promoting antioxidizing efficacy at systemic level.

As a consequence, the nutriosomes of the present invention, whether as a dispersion in a substantially water-based solvent or in lyophilized solid state, have proven to be particularly useful for the preparation of nutraceutical and/or pharmaceutical compositions for use in the treatment of forms of gastrointestinal tract dysbiosis and for fighting oxidative stress in cellular tissues.

### List of Bibliographic References

Benedetti, S., Rinalducci, S., Benvenuti, F., Francogli, S., Pagliarani, S., Giorgi, L., Micheloni, M., D'Amici, G.M., Zolla, L., Canestrari, F., 2006. Purification and characterization of phycocyanin from the blue-green alga Aphanizomenon flos-aquae. J. Chromatogr. B. Analyt. Technol. Biomed. Life Sci. 833, 12-8. doi:10.1016/j.jchromb.2005.10.010
Caddeo, C., Chessa, M., Vassallo, A., Pons, R., Diez-Sales, O., Fadda, A.M., Manconi, M., 2013. Extraction, purification and nanoformulation of natural phycocyanin (from Klamath algae) for dermal and deeper soft tissue delivery. J. Biomed. Nanotechnol. 9, 1929-1938. doi:10.1166/jbn.2013.1741
Castangia, I., Nácher, A., Caddeo, C., Merino, V., Díez-Sales, O., Catalán-Latorre, A., Fernàndez-Busquets, X., Fadda, A.M., Manconi, M., 2015. Therapeutic efficacy of quercetin enzyme-responsive nanovesicles for the treatment of experimental colitis in rats. Acta Biomater. 13, 216-27. doi:10.1016/j.actbio.2014.11.017
Manca, M.L., Marongiu, F., Castangia, I., Catalán-Latorre, A., Caddeo, C., Bacchetta, G., Ennas, G., Zaru, M., Fadda, A.M., Manconi, M., 2016. Protective effect of grape extract phospholipid vesicles against oxidative stress skin damages. Ind. Crops Prod. 83, 561-567. doi:10.1016/j.indcrop.2015.12.069
Manconi, M., Marongiu, F., Castangia, I., Manca, M.L., Caddeo, C., Tuberoso, C.I.G., D'hallewin, G., Bacchetta, G., Fadda, A.M., 2016. Polymer-associated liposomes for the oral delivery of grape pomace extract. Colloids Surfaces B Biointerfaces 146, 910-917. doi:10.1016/j.colsurfb.2016.07.043

## Claims

1. A complex of stable lamellar vesicles, comprising:
at least one phospholipid;
at least one water-soluble and digestible mixture of dextrin fibers derived from wheat and maize ;
at least one substance of natural origin having antioxidant properties; and
wherein the dextrin fibers are located both within the vesicles and in the intervesicular aqueous liquid; and
wherein said complex is dispersed or not in a substantially or totally water-based hydrophilic solvent; and wherein said dispersion has the composition of the following Table 1a):
| **Phospholipid Component** (weight/volume %) | **Dextrin Fiber** (weight/volume %) | **Antioxidant Active** (weight/volume %) | **Additional Polymer** (weight/volume %) |
|---|---|---|---|
| **1-50** | **1-60** | **0.1 - 15** | **0-20** |

2. The complex according to claim 1, further comprising an additional polymer having the function of interacting with phospholipids and dextrin fibers, thus rendering the formulation gastro-resistant.

3. The complex according to claims 1-2, wherein said at least one phospholipid is selected from the group consisting of soy or egg lecithin, phosphatidylcholine at different degrees of purity, whether hydrogenated or non-hydrogenated, phosphatidyl-ethanolamine, phosphatidylserine, phosphatidylglycerol, phosphatidylinositol, dimyristoylphosphatidylcholine, dipalmitoylphosphatidylcholine, distearoyl-phosphatidylcholine, palmitoylstearoylphosphatidylcholine, sphingomyelin, mixtures of phospholipids, hydrogenated and non-hydrogenated, and/or mixtures of phospholipids, mixtures of phospholipids prevalently containing soy phosphatidylcholine.

4. The complex according to any one of the preceding claims, wherein said antioxidant substance is selected from natural active principles, whether pure or as a mixture, or in the form of a purified or non-purified extract, having antioxidant properties, selected from ascorbic acid or prevalently polyphenols, such as gallic acid, ellagic acid, resveratrol, baicalin, quercetin, curcumin; complex macromolecular substances, such as phycocyanins; vegetable extracts containing different antioxidizing substances, such as grape extracts, vinasse and grape seed extracts, Citrus leaf and fruit extracts .

5. The complex according to any one of the preceding claims, wherein said additional polymer is selected from the group consisting of: biocompatible polymers, preferably gastro-resistant, semi-synthetic derivatives of cellulose, hydroxypropyl methylcellulose, copolymers of methacrylic acid and of the methyl ester thereof, xanthan gum, gum arabic, tragacanth gum, alginate, hyaluronic acid.

6. The complex according to any one of the preceding claims, wherein said dispersing hydrophilic solvent is water or a mixture of water and alcohols or water-soluble polyalcohols, wherein said alcohols are present in a quantity not exceeding 50% in volume.

7. The complex according to any one of the preceding claims, under the form of a stable lyophilized solid.

8. A method for preparing a complex according to any one of the preceding claims, comprising or consisting of the following steps:
- weighing all components in a container, while adding them in succession in any order; and letting them hydrate in said hydrophilic solvent for a time of approximately 4-8 hours at room temperature;
- subjecting the mixture of the hydrated components to sonication, by using a conventional ultrasonic sonication equipment commonly known and used in the art, sonicating said mixture with 10-100 cycles (1-10 seconds ON, 1-10 seconds OFF) and with an amplitude of 10 to 15 µm, at room temperature, to obtain the desired dispersion of the nanometric lamellar vesicles, the nutriosomes.

9. The method according to claim 8, wherein said method further comprises a step of lyophilizing said dispersion of nutriosomes, to give a solid and stable lyophilized complex of nutriosomes; and wherein said method further comprises or not a subsequent step of extemporaneous re-hydration of said lyophilized complex, to restore the dispersion of the nanometric lamellar vesicles before their use.

10. A nutraceutical and/or pharmaceutical composition comprising at least one complex according to any one of claims 1 to 7, for use in the treatment of forms of gastrointestinal dysbiosis and/or oxidative stress.

11. A composition for use according to claim 10 formulated for oral administration.

## Patentansprüche

1. Komplex von stabilen lamellaren Vesikeln, umfassend:
mindestens ein Phospholipid;
mindestens eine Mischung aus wasserlöslichen und verdaulichen Dextrinfasern, die aus Weizen und Mais stammen;
mindestens eine Substanz natürlichen Ursprungs mit antioxidativen Eigenschaften; und
wobei die Dextrinfasern sowohl innerhalb der Vesikel als auch in der wässrigen intravesikulären Flüssigkeit angeordnet sind; und
wobei der Komplex in einem im Wesentlichen oder vollständig auf Wasser basierenden hydrophilen Lösungsmittel dispergiert ist oder nicht; und wobei die Dispersion die Zusammensetzung der folgenden Tabelle 1a) aufweist:
| Phospholipid-Komponente (Gewicht/Volumen %) | Dextrinfaser (Gewicht/Volumen %) | Antioxidans aktiv (Gewicht/Volumen %) | Zusätzliches Polymer (Gewicht/Volumen %) |
|---|---|---|---|
| 1-50 | 1-60 | 0,1-15 | 0-20 |

2. Komplex nach Anspruch 1, weiterhin enthaltend ein zusätzliches Polymer mit der Funktion, mit Phospholipiden und Dextrinfasern zu interagieren, wodurch die Formulierung magensaftresistent gemacht wird.

3. Komplex nach den Ansprüchen 1-2, wobei das mindestens eine Phospholipid aus der Gruppe ausgewählt ist, die aus Soja oder Ei-Lecithin, Phosphatidylcholin in unterschiedlichen Reinheitsgraden, ob oder nicht hydriert, Phosphatidylethanolamin, Phosphatidylserin, Phosphatidylglycerin, Phosphatidylinositol, Dimyristoylphosphatidylcholin, Dipalmitoylphosphatidylcholin, Distearoylphosphatidylcholin, Palmitoylstearoylphosphatidylcholin, Sphingomyelin, Phospholipidmischungen, hydriert und nicht hydriert, und/oder Mischungen von Phospholipiden, Sojaphosphatidylcholin überwiegend enthaltende Phospholipidmischungen.

4. Komplex nach einem der vorhergehenden Ansprüche, wobei die antioxidative Substanz aus natürlichen Wirkstoffen, ob rein oder als Mischung, oder in Form eines gereinigten Extrakts oder nicht, mit antioxidativen Eigenschaften ausgewählt ist, aus Ascorbinsäure ausgewählt, hauptsächlich Polyphenole, wie Gallussäure, Hellaginsäure, Resveratrol, Baicalyn, Quercetin, Curcumin, komplexe makromolekulare Substanzen wie Phycocyanin und/oder Pflanzenextrakte mit verschiedenen antioxidativen Substanzen, wie Traubenextrakte, Vinasse und Traubenkernextrakte, Zedernblatt- und Fruchtextrakte.

5. Komplex nach einem der vorhergehenden Ansprüche, wobei das zusätzliche Polymer ausgewählt ist aus der Gruppe bestehend aus: biokompatible Polymere, vorzugsweise magensaftresistente, halbsynthetische Cellulosederivate, Hydroxypropylmethylcellulose, Copolymere von Methacrylsäure und seinem Methylester, Xanthangummi, Gummiarabikum, Tragant, Alginat, Hyaluronsäure.

6. Komplex nach einem der vorhergehenden Ansprüche, wobei das hydrophile Dispergier-Lösungsmittel Wasser oder eine Mischung aus Wasser und wasserlöslichen Alkoholen oder Polyalkoholen ist, wobei die Alkohole in einer Menge von nicht mehr als 50 Vol.-% vorhanden sind.

7. Komplex nach einem der vorhergehenden Ansprüche, in Form eines stabilen lyophilisierten Feststoffs.

8. Verfahren zur Herstellung eines Komplexes nach einem der vorhergehenden Ansprüche, umfassend oder bestehend aus den folgenden Schritten:
- Wiegen allen Komponenten in einem Behälter und fügen sie nacheinander in beliebiger Reihenfolge hinzu; und Hydratisieren lassen in dem hydrophilen Lösungsmittel für einen Zeitraum von etwa 4 bis 8 Stunden bei Raumtemperatur;
- Beschallen der Mischung aus hydratisierten Komponenten unter Verwendung herkömmlicher Ultraschall-Beschallungsgeräte, die allgemein bekannt und in der Technik verwendet werden, Beschallen der Mischung mit 10-100 Zyklen (1-10 Sekunden EIN, 1-10 Sekunden AUS) und mit einer Amplitude von 10 bis 15 µm bei Raumtemperatur, um die gewünschte Dispersion der nanometrischen lamellaren Vesikel, den Nutriosomen, zu erhalten.

9. Verfahren nach Anspruch 8, wobei das Verfahren ferner einen Schritt des Lyophilisierens der Dispersion von Nutriosomen umfasst, um einen festen und stabilen lyophilisierten Komplex von Nutriosomen bereitzustellen; und wobei das Verfahren ferner einen nachfolgenden Schritt der improvisierten Rehydratisierung des lyophilisierten Komplexes umfasst oder nicht, um die Dispersion der nanometrischen lamellaren Vesikel vor ihrer Verwendung wiederherzustellen.

10. Nutrazeutische und/oder pharmazeutische Zusammensetzung, enthaltend mindestens einen Komplex nach einem der Ansprüche 1 bis 7, zur Verwendung bei der Behandlung von Formen von Magen-Darm-Dysbiose und/oder oxidativem Stress.

11. Zusammensetzung zur Verwendung nach Anspruch 10, formuliert zur oralen Verabreichung.

## Revendications

1. Complexe de vésicules lamellaires stables, comprenant:
au moins un phospholipide;
au moins un mélange de fibres de dextrine hydrosolubles et digestibles dérivé du blé et du maïs;
au moins une substance d'origine naturelle ayant des propriétés anti-oxydantes; et
dans lequel les fibres de dextrine sont disposées toutes les deux à l'intérieur des vésicules et
dans le liquide aqueux intra-vésiculaire; et
dans lequel ledit complexe est dispersé ou non dans un solvant hydrophile sensiblement ou
complètement à base d'eau; et dans lequel ladite dispersion a la composition de la table 1a) suivante :
| Composant phospholipide (poids/volume %) | Fibre de dextrine (poids/volume %) | Antioxydant actif (poids/volume %) | Polymère additionnel (poids/volume %) |
|---|---|---|---|
| 1-50 | 1-60 | 0,1-15 | 0-20 |

2. Complexe selon la revendication 1, comprenant en outre un polymère additionnel ayant pour fonction d'interagir avec des phospholipides et des fibres de dextrine, rendant ainsi la formulation gastro-résistante.

3. Complexe selon les revendications 1-2, dans lequel ledit au moins un phospholipide est choisi dans le groupe constitué par lécithine de soja ou d'oeuf, phosphatidylcholine à différents degrés de pureté, qu'elle soit hydrogénée ou non, phosphatidyl-éthanolamine, phosphatidylsérine, phosphatidylglycérol, phosphatidylinositol, dimyristoylphosphatidylcholine, dipalmitoylphosphatidylcholine, distéaroyl-phosphatidylcholine, palmitoylstéaroylphosphatidylcholine, sphingomyéline, mélanges de phospholipides, hydrogénés et non hydrogénés, et/ou mélanges de phospholipides, mélanges de phospholipides majoritairement contenant de la phosphatidylcholine de soja.

4. Complexe selon l'une quelconque des revendications précédentes, dans lequel ladite substance antioxydante est choisie parmi les principes actifs naturels, qu'ils soient purs ou en mélange, ou sous forme d'extrait purifié ou non, ayant des propriétés antioxydantes, choisi parmi acide ascorbique, majoritairement polyphénols, comme acide gallique, acide hellagique, resveratrol, baicalyne, quercetyne, curcumine, substances macromoléculaires complexes comme phycocyanine et/ou extraits végétaux contenant des substances antioxydantes différentes, comme extraits de raisin, vinasses et extraits de pépins de raisin, feuille de cèdre et extraits de fruits.

5. Complexe selon l'une quelconque des revendications précédentes, dans lequel ledit polymère additionnel est choisi dans le groupe constitué par: polymères biocompatibles, de préférence gastro-résistants, dérivés semi-synthétiques de la cellulose, hydroxypropylméthylcellulose, copolymères de l'acide méthacrylique et du son ester méthylique, gomme xanthane, gomme arabique, gomme adragante, alginate, acide hyaluronique.

6. Complexe selon l'une quelconque des revendications précédentes, dans lequel ledit solvant hydrophile dispersant est de l'eau ou un mélange d'eau et d'alcools ou de polyalcools hydrosolubles, dans lequel lesdits alcools sont présents en une quantité n'excédant pas 50% en volume.

7. Complexe selon l'une quelconque des revendications précédentes, sous forme d'un solide lyophilisé stable.

8. Procédé de préparation d'un complexe selon l'une quelconque des revendications précédentes, comprenant ou consistant en les étapes suivantes:
- peser tous les composants dans un conteneur, en les ajoutant successivement dans n'importe quel ordre; et les laisser s'hydrater dans ledit solvant hydrophile pendant une durée d'environ 4 à 8 heures à température ambiante;
- soumettre le mélange des composants hydratés à une sonication, en utilisant un équipement de sonication à ultrasons conventionnel communément connu et utilisé dans l'art, sonication dudit mélange avec 10-100 cycles (1-10 secondes ON, 1-10 secondes OFF) et avec une amplitude de 10 à 15 µm, à température ambiante, pour obtenir la dispersion souhaitée des vésicules lamellaires nanométriques, les nutriosomes.

9. Procédé selon la revendication 8, dans lequel ledit procédé comprend en outre une étape de lyophilisation de ladite dispersion de nutriosomes, pour donner un complexe lyophilisé solide et stable de nutriosomes; et dans lequel ledit procédé comprend en outre ou non une étape ultérieure de réhydratation extemporanée dudit complexe lyophilisé, pour restaurer la dispersion des vésicules lamellaires nanométriques avant leur utilisation.

10. Composition nutraceutique et/ou pharmaceutique comprenant au moins un complexe selon l'une quelconque des revendications 1 à 7, pour son utilisation dans le traitement des formes de dysbiose gastro-intestinale et/ou de stress oxydatif.

11. Composition pour une utilisation selon la revendication 10, formulée pour une administration orale.
